# EUROPÄISCHE PATENTSCHRIFT

(11) **EP 4 009 919 B1**
(45) Veröffentlichungstag und Bekanntmachung des Hinweises auf die Patenterteilung: **07.01.2026**
(21) Anmeldenummer: 20745131.1
(22) Anmeldetag: 21.07.2020
(51) Int. Cl.: A61F 2/90, A61B 17/12, A61F 2/82, A61F 2/966

(54) **MEDIZINISCHES SET SOWIE MEDIZINISCHES SYSTEM ZUR BEHANDLUNG VON ANEURYSMEN**
MEDICAL KIT AND MEDICAL SYSTEM FOR THE TREATMENT OF ANEURYSMS
KIT MÉDICAL ET SYSTÈME MÉDICAL DESTINÉS AU TRAITEMENT D'ANÉVRISMES

(30) Priorität: 09.08.2019 DE 102019121546
(43) Veröffentlichungstag der Anmeldung: 15.06.2022
(73) Patentinhaber: Acandis GmbH, 75177 Pforzheim (DE)
(72) Erfinder: BÜCHERT, Michael, 75177 Pforzheim (DE); SCHÖNBERGER, Eugen, 75176 Pforzheim (DE); CATTANEO, Giorgio, 76199 Karlsruhe (DE)
(74) Vertreter: Meissner Bolte Partnerschaft mbB
(86) Internationale Anmeldenummer: PCT/EP2020/070499
(87) Internationale Veröffentlichungsnummer: WO 2021/028160

(56) Entgegenhaltungen:
- DE-A1- 102010 035 543
- DE-A1- 102011 011 869
- US-A1- 2011 319 917
- US-A1- 2018 193 026

## Beschreibung

Die Erfindung betrifft ein medizinisches Set zur Behandlung von Aneurysmen. Ferner betrifft die Erfindung ein medizinisches System.

WO 2014/177634 A1 beschreibt einen hochflexiblen Stent, der eine komprimierbare und expandierbare Gitterstruktur aufweist, wobei die Gitterstruktur einstückig ausgebildet ist. Die Gitterstruktur umfasst geschlossene Zellen, die durch jeweils vier Gitterelemente begrenzt sind. Die Gitterstruktur weist wenigstens einen Zellenring auf, der zwischen drei und sechs Zellen umfasst.

Aus US 2018/193 026 A1, US 2011/319 917 A1 und DE 10 2011 896 A1 sind jeweils Vorrichtungen mit Gitterstrukturen bekannt, die zur Behandlung von Aneurysmen geeignet sind, jedoch unterschiedliche Schwerpunkte hinsichtlich ihrer technischen Augestaltung und des damit erzielten Zwecks setzen.

Aus der Praxis der Anmelderin sind außerdem Stents mit Gitterstrukturen bekannt, die aus einem einzigen Draht gebildet sind. Der Draht ist mit sich selbst verflochten, um ein röhrchenförmiges Geflecht zu bilden. An den axialen Enden des röhrchenförmigen Geflechts ist der Draht umgelenkt, so dass sich atraumatisch wirkende Schlaufen bilden. Die axialen Enden können trichterförmig ausgeweitet sein.

Die bekannte medizinische Vorrichtung eignet sich insbesondere zur Behandlung von Aneurysmen in kleinen, zerebralen Blutgefäßen. Derartige Blutgefäße weisen einen sehr kleinen Querschnittsdurchmesser auf und sind oft stark gewunden. Der bekannte Stent ist dazu hochflexibel gestaltet, so dass er einerseits auf einen sehr kleinen Querschnittsdurchmesser komprimierbar ist und andererseits eine hohe Biegeflexibilität aufweist, die die Zuführung in kleine zerebrale Blutgefäße ermöglicht.

Zur Behandlung von Aneurysmen in zerebralen Blutgefäßen ist es zweckmäßig, Stents einzusetzen, die sich über ein Aneurysma spannen und dieses vom Blutfluss innerhalb des Blutgefäßes abschirmen. Um dies zu ermöglichen, ist es bekannt, Stents mit einer Abdeckung zu versehen, die die Zellen des Stents verschließt und so einen Blutstrom in ein Aneurysma verhindert.

Eine weitere ergänzende oder alternative Behandlungsmethode von Aneurysmen ist die Implantation sogenannter Coils in das Aneurysma, welche dort zu einer Blutgerinnung führen. Der resultierende Thrombus verhindert dann die Blutzirkulation im Aneurysma und somit das Risiko einer Ruptur mit anschließender Blutung.

Besonders bei breithalsigen Aneurysmen neigen die Coils jedoch dazu, während der Implantation in die Blutbahn zu wandern und somit einen Verschluss des Hauptgefäßlumen zu verursachen. Bei der Technik "Ballon assisted Coiling" werden Katheter mit "Compliance-Ballonen" im Gefäß, speziell an der Höhe des Aneurysmahalses, positioniert. Der mit Kontrastmittel gefüllte Ballon schließt den Aneurysmahals während der Platzierung der Coils und zwingt die Coils in eine kompakte Anordnung innerhalb des Aneurysma-Raums. Weil Coils plastisch verformbar sind, bleiben diese dann in ihrer Form. Sie verlassen das Aneurysma auch dann nicht, wenn der Ballon entfernt wird. Ein Problem besteht jedoch hierbei insbesondere aufgrund der Tatsache, dass der Ballon das Gefäß verschließt. Bei anhaltender Prozedur (bei großen Aneurysmen werden mehrere Coils platziert, die Prozedur kann mehrere Minuten dauern), wird die Blutströmung in der Zeit vollständig unterbrochen. Zwar sorgen Kollateralgefäße für eine Versorgung von nachgelagertem Gewebe, trotzdem bleibt die Gefahr einer Unterperfusion bestehen.

Weiterhin wird während der Prozedur ein Katheter, durch den die Coils geführt werden, an der Seite des Ballons "gejailt", das heißt festgeklemmt. Bei der evtl. Notwendigkeit, Coil-Katheter zu wechseln (z.B im Fall einer Beschädigung) bei noch nicht vollständiger Prozedur, soll der Ballon deflatiert (entleert) werden, um den Katheter zurückziehen zu können. In dieser Phase kann es zu einer Verschiebung der Coils, die noch nicht vollständig und kompakt in dem Gefäß liegen, kommen. Diese können dann zu einem Gefäßverschluss führen.

Vor diesem Hintergrund ist es Aufgabe der Erfindung, ein medizinisches Set zur Behandlung von Aneurysmen anzugeben, mit dessen Hilfe die Gefahr von Gefäßverschlüssen zumindest reduziert ist. Eine weitere Aufgabe der Erfindung besteht darin, ein medizinisches System anzugeben.

Erfindungsgemäß wird diese Aufgabe im Hinblick auf das medizinische Set durch den Gegenstand des Patentanspruchs 1 und im Hinblick auf das medizinische System durch den Gegenstand des Patentanspruchs 8 gelöst.

Bevorzugte Ausgestaltungen, Weiterbildungen und Varianten sind Gegenstand der Unteransprüche.

Konkret wird die Aufgabe gelöst durch ein medizinisches Set zur Behandlung von Aneurysmen mit einem Hauptkatheter sowie mit einer durch den Hauptkatheter hindurch an einen Behandlungsort bewegbaren Abdeckvorrichtung. Unter dem Behandlungsort kann hierbei eine Stelle entlang eines Gefäßes verstanden werden, an der das Aneurysma ausgebildet ist. Die Abdeckvorrichtung dient hierbei einem temporären Abdecken des Aneurysmas, wobei die Abdeckvorrichtung eine selbstexpandierbare Gitterstruktur umfasst.

Die Gitterstruktur weist einen zylinderförmigen Abschnitt auf, der an einem distalen Längsende offen und wenigstens teilweise mit einer Abdeckung versehen ist. Unter dem distalen Längsende kann hierbei das vom Hauptkatheter abgewandte Ende des zylinderförmigen Abschnitts verstanden werden. Weiterhin weist die Gitterstruktur einen trichterförmigen Abschnitt auf, der dauerhaft mit einem innerhalb des Hauptkatheters verschiebbaren Transportdraht verbunden und über eine gesamte Umfangsfläche abdeckungsfrei ist. Unter der Umfangsfläche kann hierbei eine Mantelfläche des trichterförmigen Abschnitts verstanden werden, sodass durch diese abdeckungsfreie Ausgestaltung die Gitterstruktur in einem expandierten Zustand der Gitterstruktur längsaxial blutdurchströmbar ist. Unter dem trichterförmigen Abschnitt kann hierbei zum einen ein rotationssymmetrischer trichterförmiger Abschnitt aber auch alternativ ein nicht rotationasymmetrischer trichterförmiger Abschnitt, beispielsweise in Form einer nicht konzentrischen Verbindungsstelle verstanden werden. Die nicht konzentrische Verbindungsstelle kann hierbei beispielsweise auf der Mantelfläche angeordnet sein. Weiterhin alternativ kann der trichterförmige Abschnitt lediglich durch einzelne Drähte, vorzugsweise durch zwei bis sechs Drähte und speziell aus genau zwei Drähten, der Gitterstruktur gebildet sein, die von Schlaufen beziehungsweise den letzten Zellen der Gitterstruktur abgehen. Somit ist in diesem Fall keine geschlossene Gitterstruktur ausgebildet. In diesem Fall kann eine Anbindung der Gitterstruktur an einen Transportdraht auch umfangsseitig an einer Kante der Gitterstruktur erfolgen, indem beispielsweise ein oder mehrere Drähte eine Verlängerung ausbilden oder verlängert sind, die mit dem Transportdraht verbunden ist. Eine derartige Ausgestaltung ist beispielsweise aus der Offenlegungsschrift DE 10 2009 056 450 A1, die auf die Anmelderin zurückgeht, zu entnehmen, auf die insoweit Bezug genommen wird. Somit ist eine vollständige Durchströmbarkeit der Gitterstruktur erreicht, da selbst die Anbindung an den Transportdraht im Wesentlichen parallel zu der Gefäßwand und an der Gefäßwand des Gefäßes entlang angeordnet ist.

Weiterhin kann der trichterförmige Abschnitt auch eine gestufte Verjüngungskontur aufweisen. Unter längsaxial blutdurchströmbar kann hierbei verstanden werden, dass das durch das Gefäß fließende Blut durch die Ausgestaltung der Abdeckvorrichtung nicht wesentlich im Fluss gestört wird.

Die Gitterstruktur weist zweckdienlicherweise eine Formgedächtnislegierung, insbesondere Nitinol, auf oder ist bevorzugt aus einem derartigen Material gebildet. Die Formgedächtnislegierung ist zur Ausbildung der Gitterstruktur vorzugsweise lasergeschnitten.

Der Vorteil hierbei ist, dass durch die längsaxiale Blutdurchströmbarkeit der Gitterstruktur eine Blutströmung im Gefäß während des Setzens eines Coils gewährleistet ist. Wegen der längsaxial durchströmbaren Gitterstruktur wird vorteilhaft ein Blutfluss in Längsrichtung durch das Blutgefäß kaum behindert, jedoch eine Einströmung des Blutes in ein abzweigendes Aneurysma durch die Abdeckung unterbunden oder zumindest ein Strömungseinfluss in das Aneurysma reduziert. Ein mögliches und unerwünschtes Verschließen des Gefäßes, wie es beispielsweise bei der eingangs genannten Technik mittels des Ballons auftreten kann, ist somit zumindest reduziert und vorzugsweise ausgeschlossen.

Erfindungsgemäß ist die Abdeckung als eine elektrogesponnene Abdeckung ausgebildet.

Bei einem elektrogesponnenen Gewebe sind Poren üblicherweise unregelmäßig gestaltet. Das Herstellungsverfahren erlaubt es jedenfalls nicht, eine musterartige Anordnung oder Gestaltung von Poren zu erstellen. Allerdings können die Porengrößen anhand der Prozessparameter zumindest soweit eingestellt werden, dass sichergestellt ist, dass wenigstens ein Teil der Poren eine gewisse Mindestgröße aufweist.

Beispielsweise kann der Prozess des Elektrospinnens unmittelbar auf der Gitterstruktur erfolgen, so dass bei der Bildung der Abdeckung gleichzeitig eine Verbindung mit der Gitterstruktur hergestellt wird. Die Abdeckung kann mit der Gitterstruktur stoffschlüssig verbunden sein. Beispielsweise kann die Abdeckung mit der Gitterstruktur durch eine Klebeverbindung verbunden sein. Die Klebeverbindung kann durch einen Haftvermittler hergestellt werden. Der Haftvermittler kann beispielsweise Polyurethan umfassen oder daraus bestehen.

Die Abdeckung aus einem elektrogesponnenen Gewebe ist darüber hinaus äußerst dünn und flexibel, was die Flexibilität der Gitterstruktur nur unwesentlich bis nicht beeinträchtigt. Insbesondere hindert die Abdeckung die Gitterstruktur im Unterschied zu vorbekannten Abdeckungen, die aus Textilmaterialien hergestellt sind, kaum an der Komprimierung. Insgesamt lässt sich also die gesamte Abdeckvorrichtung auf einen erheblich kleineren Querschnittsdurchmesser komprimieren und so über kleine Katheter in besonders kleine Blutgefäße führen. Dies ist insbesondere für die Behandlung von Aneurysmen in zerebralen Blutgefäßen relevant, wozu sich die Erfindung besonders eignet.

Mit dem erfindungsgemäßen medizinischen Set sind daher auch Behandlungen in Blutgefäßen möglich, die mit bisherigen medizinischen Vorrichtungen, die eine Gitterstruktur und eine Abdeckung aufweisen, nicht erreicht werden können. Wegen der hohen Komprimierbarkeit der erfindungsgemäßen Vorrichtung treten bei der Zuführung durch einen Katheter sehr geringe Zuführkräfte auf. Insbesondere können die Zuführkräfte bei der Vorrichtung mit Abdeckung im Vergleich zur Zuführung der Gitterstruktur allein gleich oder kleiner sein.

Weiterhin kann durch die Abdeckvorrichtung, welche zweckdienlicherweise am Behandlungsort, also auf Höhe des Aneurysmas angeordnet ist, beim und nach dem Setzen der Coils ein Herauswandern der Coils aus dem Aneurysma verhindert werden, sodass die Gefahr eines durch die Coils verursachten Gefäßverschlusses ebenfalls zumindest stark reduziert und vorzugsweise ausgeschlossen werden kann.

Um eine ausreichende Flexibilität der Abdeckung zu gewährleisten, ist diese vorzugsweise aus unregelmäßig netzartig angeordneten Fäden gebildet, die eine Fadendicke zwischen 0,1 µm und 3 µm, insbesondere zwischen 0,2 µm und 2 µm, insbesondere zwischen 0,5 µm und 1,5 µm, insbesondere zwischen 0,8 µm und 1,2 µm, aufweisen.

In einer Ausführungsform ist die Abdeckung porös und insbesondere blutdurchlässig. Unter porös kann hierbei verstanden werden, dass die Abdeckung netzartig oder als ein Netz ausgebildet ist. Dieser Ausführungsform liegt der Gedanke zugrunde, sich im Bereich der Abdeckung befindliche Zellen mit Blut und somit mit den bereits erwähnten Nährstoffen versorgt werden können, sodass hierdurch keine Unterversorgung beim Setzen der Coils erfolgt. Weiterhin liegt dieser Ausführungsform der Gedanke zugrunde, durch die Abdeckung des Aneurysmas die Strömungsgeschwindigkeit des Blutes und somit ein Pulsieren des Blutes, welches durch das Gefäß fließt, zumindest zu reduzieren, weil dieses Pulsieren ein Setzen der Coils erschweren würde.

Das erfindungsgemäße medizinische Set ermöglicht somit eine gute Abschirmung eines Aneurysmas zum Zurückhalten der in das Aneurysma eingesetzten Coils, lässt jedoch gleichzeitig eine Nährstoffzufuhr in das Aneurysma zu. Auch die Nährstoffzufuhr in abzweigende Blutgefäße und angrenzende Gefäßinnenwände wird durch das medizinische Set erreicht. Die Abdeckung, die aus dem elektrogesponnenen Gewebe gebildet ist, ermöglicht eine Abdeckung eines Aneurysmas, lässt jedoch zeitgleich eine gewisse Durchlässigkeit für Blut zu. Diese Durchlässigkeit ist zweckmäßig, um die Zellen der Aneurysmenwand mit Nährstoffen zu versorgen. Dadurch wird eine Degeneration der Zellen und eine daraus möglicherweise resultierende Ruptur des Aneurysmas vermieden.

Gemäß einer alternativen Ausführungsform ist die Abdeckung insbesondere geringfügig porös oder dicht und somit blutundurchlässig. Insbesondere kann somit trotz der geringfügigen Porosität, die aus dem Elektrospinnprozess resultiert, die Abdeckung so dicht sein, dass das Blut an dieser vorbeiströmt und somit beispielsweise eine Einströmung von Blut in das Aneurysma verhindert wird. Das Blut strömt hierbei vielmehr an der Abdeckung des Aneurysmas vorbei.

Zweckdienlicherweise weist die Abdeckung eine Porosität von höchstens 70%, und insbesondere von höchstens 50% auf. Hierdurch wird die Stabilität der Abdeckung zum einen in Bezug auf eine Kraft der Coils erhöht, welche beispielsweise beim im Aneurysma eingesetzten Zustand, auf die Abdeckung drückt. Zum anderen wird die Stabilität der Abdeckung im Hinblick auf eine Bruchstabilität vorteilhaft optimiert. Außerdem führt eine derart niedrigporöse Abdeckung zu einer höheren, insbesondere temporären, Verlangsamung der Blutströmung im Bereich der Abdeckung, was während der Platzierung der Coils von Vorteil ist. Das Aneurysma pulsiert hierdurch während der Platzierung der Coils weniger und ermöglicht somit eine schnellere und genauere Platzierung der Coils.

In einer Ausführungsform weist die Abdeckung eine Porosität von wenigstens 5%, insbesondere von wenigstens 10%, insbesondere von wenigstens 20%, insbesondere von wenigstens 30%, insbesondere von wenigstens 40% und insbesondere von wenigstens 45% auf. Dieser Ausführungsform liegt der Gedanke zugrunde, dass durch eine bestehende Porosität in Höhe der zuvor genannten Prozentangaben - wie bereits vorstehend erwähnt - eine Versorgung von beispielsweise Seitengefäßen während der Platzierung der Coils gewährleistet ist.

Außerdem ist die Abdeckung aufgrund ihrer Porosität speziell für Mikrokatheter geeignet, da die Abdeckung komprimierbar ist und somit mit niedrigeren Reibungskräften durch die Mikrokatheter an den Behandlungsort einführbar und auch zurückführbar ist. Unter Mikrokatheter kann hierbei ein Katheter verstanden werden, der einen Durchmesser mit einem Wert im Bereich von 0,3mm bis 0,75mm aufweist.

Gemäß einer zweckdienlichen Ausgestaltung erstreckt sich die Abdeckung über die gesamte Umfangsfläche des zylinderförmigen Abschnitts. Hierdurch werden die zuvor genannten Vorteile, insbesondere hinsichtlich einer Stabilität der Abdeckung und somit der Gitterstruktur vorteilhaft optimiert.

Gemäß einer zweckdienlichen Weiterbildung erstreckt sich die Abdeckung über einen Teil, insbesondere über höchstens 50%, insbesondere über höchstens 40% und insbesondere über höchstens 30% des Umfangs des zylinderförmigen Abschnitts. Bevorzugt erstreckt sich somit die Abdeckung somit lediglich über den Behandlungsort, also beispielsweise über eine Öffnung des Aneurysmas. Durch diese Weiterbildung wird sichergestellt, dass zum einen eine Öffnung des Aneurysmas zum Fixieren der darin platzierten Coils ausreichend verschlossen ist. Zum anderen ist hierdurch sichergestellt, dass insbesondere sich auf Höhe des Aneurysmas befindliche Zellen und/oder Seitengefäße durch die fehlende Abdeckung besser und weiterhin mit Blut und somit mit Nährstoffen versorgt werden können.

Gemäß einer ergänzenden oder alternativen Ausführungsform erstreckt sich die Abdeckung bei mindestens 80 %, insbesondere über mindestens 90 % und insbesondere über 100 % der Länge des zylinderförmigen Abschnitts. Diese Ausführungsform ist insbesondere geeignet für fusiforme bzw. langhalsige Aneurysmen, in denen ein längerer Abschnitt abgedeckt werden soll. Unter fusiformen Aneurysmen werden hierbei Aneurysmen verstanden, die sich über wenigstens 50%, insbesondere über wenigstens 75% des gesamten Umfangs oder über den gesamten Umfang eines Blutgefäßes erstrecken.

In einer Ausführungsform erstreckt sich die Abdeckung über höchstens 80%, insbesondere über höchstens 60%, insbesondere über höchstens 40% der Länge des zylinderförmigen Abschnitts, wobei die Abdeckung vom distalen Ende des zylinderförmigen Abschnitts beabstandet ist. Durch die hiermit nicht vollständige Abdeckung des zylindrischen Bereichs der Gitterstruktur ist gewährleistet, dass eventuelle Gefäße in der Nähe des Aneurysmas und speziell in der Nähe der Öffnung des Aneurysmas weiterhin perfundiert werden, also mit Blut versorgt werden. Diese Ausführungsform hat sich ebenfalls besonders bei Aneurysmen mit benachbarten Seitenästen sowie bei kleineren Aneurysmen als geeignet erwiesen.

Erfindungsgemäß weist die Abdeckung auf einer Fläche von 100.000 µm² mindestens 10 Poren auf, die eine Größe von mindestens 15 µm² aufweisen. Im Zuge der Herstellung der Abdeckung lässt sich die Mindestgröße der Poren insbesondere durch die Prozessdauer des Elektrospinnens einstellen. Diese Kombination aus einer bestimmten Mindestanzahl an Poren und einer Mindestgröße dieser Poren hat sich in der Praxis als besonders förderlich für eine ausreichende Blutdurchlässigkeit der Abdeckung bei gleichzeitig guter Abdeckwirkung gezeigt.

Bevorzugt ist die Abdeckung durch ein Kunststoffmaterial, insbesondere durch ein Polymer, und vorzugsweise durch Polyurethan gebildet. Derartige Materialien sind besonders leicht und lassen sich gut in feinen Fäden durch ein elektrospinnendes Verfahren herstellen. Das Kunststoffmaterial ermöglicht es also einerseits eine besonders dünne und feinporige Abdeckung herzustellen. Andererseits weist das Kunststoffmaterial in sich bereits eine hohe Flexibilität auf, so dass eine hohe Komprimierbarkeit des medizinischen Sets erreicht wird. Alternativ kann die Abdeckung auch durch Polyethylen, durch Flurpolymere oder durch beispielsweise auf Polycarbonat basierenden, thermoplastischen Polyurethanen gebildet sein. Weiterhin kann beispielsweise alternativ oder ergänzend vorgesehen sein, dass Füllstoffe, wie z.B. anti-thrombogene Stoffe in die zuvor genannten Materialien der Abdeckung eingebettet sind, bevor mit diesen durch den Elektrospinning-Prozess die Abdeckung gebildet wird. Alternativ oder ergänzend ist die Abdeckung mit derartigen Stoffen, beispielsweise mit antithrombogenen Stoffen beschichtet. Hierzu ist dann die Oberfläche der Abdeckung insbesondere mit einer Nanobeschichtung versehen.

In einer weiteren Ausführungsform ist die Abdeckung auf einer Außenseite und/oder auf eine Innenseite der Gitterstruktur angeordnet. Die Gitterstruktur bildet in einer Konstellation, in der die Abdeckung auf der Außenseite der Gitterstruktur angeordnet ist eine Trägerstruktur, die eine ausreichende Radialkraft aufbringt, um die Abdeckung gegen eine Gefäßwand zu fixieren. Die Gitterstruktur stützt insoweit die außen angeordnete Abdeckung.

Alternativ oder zusätzlich kann die Abdeckung auf einer Innenseite der Gitterstruktur angeordnet sein. Insbesondere ist es möglich, dass die Gitterstruktur zwischen zwei Abdeckungen eingebettet ist, die jeweils durch ein elektrogesponnenes Gewebe gebildet sind. Die Gitterelemente der Gitterstruktur können insofern von dem elektrogesponnenen Gewebe vollständig ummantelt sein. Konkret kann vorgesehen sein, dass sich das elektrogesponnene Gewebe einer Abdeckung auf der Innenseite der Gitterstruktur in durch die Zellen der Gitterstruktur hindurcherstreckt und mit dem elektrogesponnenen Gewebe einer Abdeckung auf der Außenseite der Gitterstruktur verbunden ist. Die Gitterelemente, die die Zellen begrenzen, sind so auf allen Seiten von elektrogesponnenen Gewebe ummantelt.

Gemäß einer bevorzugten Ausgestaltung ist die Gitterstruktur aus Stegen gebildet, die einstückig, also monolithisch, miteinander verbunden sind und geschlossene, insbesondere rautenförmige Zellen begrenzen. Vorzugsweise weist die Gitterstruktur in Umfangsrichtung 3 bis 9, insbesondere 4 bis 6 hintereinander angeordnete Zellen auf, die einen umfangsseitigen Zellenring ausbilden.

Die Gitterstruktur kann somit grundsätzlich als einstückige Gitterstruktur ausgebildet sein. Es ist auch möglich, das die Gitterstruktur aus miteinander verflochtenen Drähten gebildet ist. Das Drahtgeflecht kann aus einem einzigen Draht bestehen, der an den Längsenden der Netzstruktur umgelenkt und zurückgeführt wird. Der Draht kann mit sich selbst verflochten werden, um die Netzstruktur zu bilden. Die Netzstruktur kann auch aus mehreren Drähten bestehen, die miteinander verflochten sind. Die mehreren Drähte können an einem axialen Längsende umgelenkt und zurückgeführt werden, während das gegenüberliegende axiale Längsende Drahtenden aufweisen kann, die offen sind. Es ist auch möglich, dass die miteinander verwebten Drähte offene Drahtenden an beiden axialen Längsenden aufweisen. Insoweit ist es in bevorzugten Ausführungsformen vorgesehen, dass die Gitterelemente Stege bilden, die durch Stegverbinder einstückig miteinander gekoppelt sind (einstückige Gitterstruktur). Der Draht kann ein röntgensichtbares Kernmaterial und ein Mantelmaterial aus einer Formgedächtnislegierung aufweisen. Insbesondere ist vorgesehen, dass das Volumenverhältnis zwischen dem Kernmaterial, vorzugsweise Platin, und dem Volumen des gesamten Verbunddrahts zwischen 20% und 40%, insbesondere zwischen 25% und 35% ist. Während eine geflochtene Gitterstruktur sich durch eine besonders hohe Flexibilität, insbesondere Biegeflexibilität, auszeichnet, weist eine einstückige Gitterstruktur eine vergleichsweise dünne Wandstärke auf, so dass die Gitterstruktur den Blutfluss innerhalb eines Blutgefäßes weniger stark beeinflusst. Die Stege weisen weiterhin bevorzugt eine Dicke mit einem Wert im Bereich zwischen 30µm und 60µm auf.Die Zellen sind weiterhin jeweils durch insgesamt vier Stege begrenzt, wobei die Grundgeometrie der Zellen im Wesentlichen im bevorzugten Design rautenförmig ist. Insbesondere ist jede Zelle durch zwei Paar Stege begrenzt, wobei die Stege, die im Wesentlichen parallel zueinander oder gegenüberliegend und nicht direkt miteinander verbunden sind, ein Stegpaar bilden. Ein derartiges Design ist bereits in der Fig. 1 und den Absätzen [0041] bis [0046] der Patentschrift DE 10 2011 009 371 B3 der Anmelderin beschrieben, auf die insoweit verwiesen wird. Die Stege eines ersten Stegpaares weisen eine geringere Stegbreite auf als die Stege eines zweiten Stegpaares. Diese Anordnung der Stege mit unterschiedlichen Stegbreiten erhöht die Flexibilität der Netzstruktur und erleichtert so die Einführung der medizinischen Vorrichtung in menschliche Gefäße, insbesondere wenn diese starke Gefäßkrümmungen aufweisen. Die erhöhte Flexibilität verbessert die Anbringung an die Gefäßwand und verhindert so die Bildung von kongestiven Bereichen, die die Thrombose fördern. Die gute Flexibilität und die daraus resultierende gute Einführbarkeit im und/oder durch den Katheter ist besonders wichtig in Kombination mit einer biologischen Beschichtung, vorzugsweise Fibrin, vorzugsweise Fibrin, vorzugsweise Fibrin einschließlich Heparin.

Ein nebengeordneter Aspekt der Erfindung betrifft ein medizinisches System zur Behandlung von Aneurysmen mit einem medizinischen Set. Bei dem Set handelt es sich konkret um das bereits vorstehend beschriebene medizinische Set, welches einen Hauptkatheter und eine durch den Hauptkatheter hindurch an einen Behandlungsort bewegbaren Abdeckvorrichtung zum temporären Abdecken eines Aneurysmas aufweist. Zusätzlich weist das medizinische System wenigstens ein Embolisationsmittel zur Platzierung im Aneurysma auf.

Zweckdienlicherweise ist weiterhin ist ein Zusatzkatheter vorgesehen, der einen proximalen Bereich, einen Mittenbereich sowie einen distalen Bereich aufweist. Der Zusatzkatheter ist vorzugsweise Teil des medizinischen Systems. Der Zusatzkatheter dient einem Einführen des Embolisationsmittels in das Aneurysma. Der Zusatzkatheter ist hierbei vom Hauptkatheter unabhängig und/oder gegenüber dem Hauptkatheter relativbeweglich.

Gemäß einer ersten Ausführungsform des medizinischen Systems ist der Mittenbereich des Zusatzkatheters innerhalb der Abdeckvorrichtung angeordnet. Der distale Bereich ist hierbei außerhalb der Abdeckvorrichtung und insbesondere im Aneurysma angeordnet. Der proximale Bereich ist hierbei parallel zum Hauptkatheter angeordnet. Mit anderen Worten wird der Zusatzkatheter gemäß dieser Ausführungsform zum Platzieren des Embolisationsmittels zunächst parallel zum Hauptkatheter geführt und dann durch die Abdeckvorrichtung hindurch, also auch durch die Abdeckung hindurch in das Aneurysma geführt, um dort das Embolisationsmittel zu platzieren.

Gemäß einer alternativen Ausführungsform des medizinischen Systems ist der proximale Bereich parallel zum Hauptkatheter angeordnet. Weiterhin ist der Mittenbereich hierbei genauso wie der distale Bereich außerhalb der Abdeckvorrichtung angeordnet, wobei das distale Ende weiterhin insbesondere im Aneurysma angeordnet ist. Mit anderen Worten ist hierbei der gesamte Zusatzkatheter im Wesentlichen parallel neben dem Hauptkatheter sowie der Abdeckvorrichtung in das Aneurysma geführt. Der Zusatzkatheter schiebt sich somit unter der Abdeckung in das Aneurysma, um dort das Embolisationsmittel zu platzieren.

Zweckdienlicherweise weisen die Zellen der Gitterstruktur im expandierten Zustand einen Inkreisdurchmesser auf oder sind auf einen Inkreisdurchmesser aufweitbar, der wenigstens dem Außendurchmesser des Zusatzkatheters entspricht. Der Inkreisdurchmesser ist der Durchmesser des größtmöglichen Kreises, der sich in die Pore einschreiben lässt. Mit anderen Worten entspricht der Inkreisdurchmesser der Pore dem Außendurchmesser eines Zylinders, der sich gerade noch durch die Pore schieben lässt. Hierdurch wird insbesondere bei der vorstehend erläuterten ersten Ausführungsform des medizinischen Systems sichergestellt, dass der Zusatzkatheter und speziell dessen Mittenbereich einfach in die Abdeckvorrichtung eingeführt werden kann.

Bevorzugt ist das Embolisationsmittel durch einen plastisch verformbaren Draht, insbesondere durch einen bereits vorstehend erwähnten Coil, oder durch eine Flüssigkeit, z.B. durch ein Hydrogel gebildet. Derartige Ausbildungen des Embolisationsmittels haben sich im Hinblick auf eine Behandlung von Aneurysmen als besonders geeignet erwiesen.

Die im Hinblick auf das medizinische Set aufgeführten Vorteile und bevorzugten Ausgestaltungen sind sinngemäß auf das medizinische System zu übertragen und umgekehrt. Alle in Bezug auf das medizinische Set und in Bezug auf das medizinische System aufgeführten Dimensionsangaben gelten für einen expandierten Zustand der Gitterstruktur, sofern nicht etwas Anderes angegeben ist.

Ausführungsbeispiele der Erfindung werden nachfolgend anhand der beigefügten, schematisierten Zeichnungen näher erläutert. Diese zeigen teilweise in stark vereinfachten Darstellungen:
- Fig.1: eine Seitenansicht eines erfindungsgemäßen medizinischen Sets gemäß einer ersten Ausführungsform,
- Fig. 2: eine Seitenansicht eines erfindungsgemäßen medizinischen Sets gemäß einer zweiten Ausführungsform,
- Fig. 3: eine Seitenansicht eines erfindungsgemäßen medizinischen Sets gemäß einer dritten Ausführungsform,
- Fig. 4: eine Seitenansicht eines erfindungsgemäßen medizinischen Systems gemäß einer ersten Ausführungsform,
- Fig. 5: eine Seitenansicht eines erfindungsgemäßen medizinischen Systems gemäß einer zweiten Ausführungsform sowie
- Fig. 6: eine Rasterelektronenmikroskopaufnahme einer Abdeckung einer Abdeckvorrichtung eines erfindungsgemäßen Sets gemäß einer bevorzugten Ausführungsform.

In den Figuren sind gleichwirkende Teile stets mit den gleichen Bezugszeichen dargestellt.

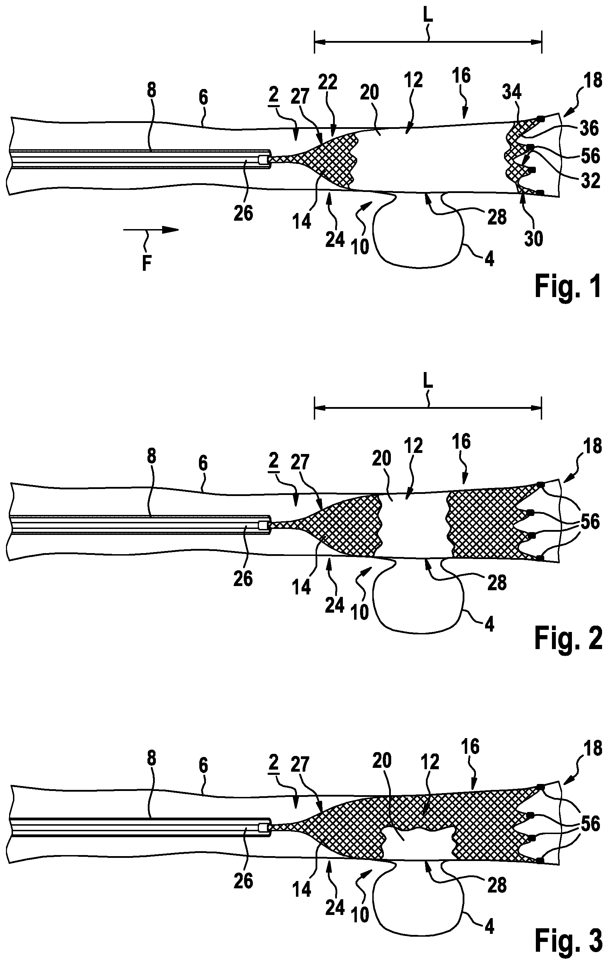

Das in Fig. 1 schematisch dargestellte medizinische Set 2 dient zur Behandlung von Aneurysmen 4 und ist in Fig. 1 in einem innerhalb eines Gefäßes 6 angeordnetem Zustand gezeigt.

Das medizinische Set 2 weist einen Hauptkatheter 8 sowie eine durch den Hauptkatheter 8 hindurch an einen Behandlungsort 10 bewegbare Abdeckvorrichtung 12 auf. Bei den Behandlungsort 10 handelt es sich vorzugsweise um den Ort entlang des Gefäßes 6, an dem das Aneurysma 4 ausgebildet ist. Die Abdeckvorrichtung 12 dient einem temporären Abdecken des Aneurysmas 4, wobei die Abdeckvorrichtung 12 eine selbstexpandierbare Gitterstruktur 14 umfasst. Die Gitterstruktur 14 ist hierzu bevorzugt aus einem Formgedächtnismaterial gefertigt. Weiterhin weist die Gitterstruktur 14 einen zylinderförmigen Abschnitt 16 auf. Der zylinderförmiger Abschnitt 16 ist an einem distalen Längsende 18 offen ausgestaltet. Weiterhin ist der zylinderförmige Abschnitt 16 wenigstens teilweise mit einer Abdeckung 20 versehen.

In Fig. 1 ist eine erste Ausführungsform des medizinischen Sets 2 gezeigt, bei der sich die Abdeckung 20 insbesondere über mindestens 90 % und insbesondere über 100 % der Länge L des zylinderförmigen Abschnitts 16 erstreckt. Zudem erstreckt sich die Abdeckung 20 im Ausführungsbeispiel gemäß Fig. 1 über den gesamten Umfang bzw. die gesamte Umfangsfläche des zylinderförmigen Abschnitt 16.

Weiterhin weist die Gitterstruktur 14 einen trichterförmigen Abschnitt 22 auf, der an einen proximalen Längsende 24 der Gitterstruktur 14, also in Richtung des Hauptkatheters 8, ausgebildet ist.

Weiterhin ist der trichterförmige Abschnitt 22 und somit die Gitterstruktur 14 dauerhaft mechanisch mit einem innerhalb des Hauptkatheters 8 verschiebbaren Transportdraht 26 verbunden. Durch diesen Transportdraht 26 ist die Gitterstruktur 14 und somit die Abdeckvorrichtung 12 in einem nicht expandierten Zustand durch den Hauptkatheter 8 schiebbar und zurückziehbar.

Der trichterförmige Abschnitt 22 ist weiterhin über eine gesamte Umfangsfläche 27 abdeckungsfrei, vorzugsweise lediglich aus der Gitterstruktur 14 gebildet, sodass in einem expandierten Zustand die Gitterstruktur 14 längsaxial, also in und entgegen einer Fließrichtung F blutdurchströmbar ist.

Die Gitterstruktur 14 ist insbesondere derart innerhalb des Gefäßes 6 angeordnet, dass die Abdeckung 20 auf einer Höhe mit einer Öffnung 28 des Aneurysmas 4 platziert ist, sodass diese von der Abdeckung 20 abgedeckt wird und gleichzeitig ein Blutfluss durch das Gefäß 6 nicht gestoppt wird. Die Abdeckung 20 ist somit im Ausführungsbeispiel gemäß Fig. 1 im Wesentlichen rohrartig, insbesondere nach Art eines Hohlzylinders, ausgebildet.

Die Abdeckung 20 ist hierbei vorzugsweise porös und blutdurchlässig ausgebildet, sodass eine Nährstoffversorgung, der von der Abdeckung 20 abgedeckten Zellen weiterhin gewährleistet ist. Alternativ kann die Abdeckung 20 jedoch auch gering porös bis dicht und somit fluiddicht und insbesondere blutundurchlässig ausgebildet sein.

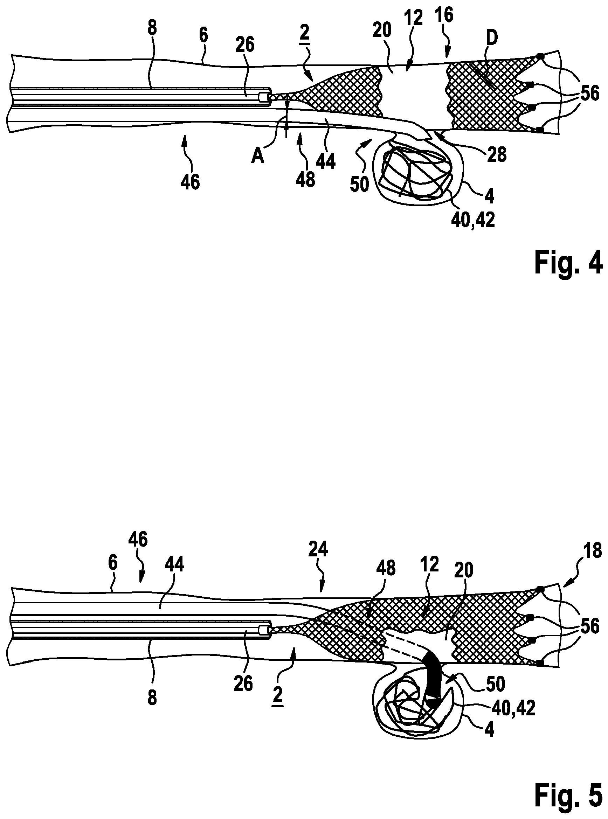

Die Abdeckung 20 dient hierbei, insbesondere bei einem Platzieren eines Embolisationsmittels 40 (vgl. Fig. 4 oder Fig. 5) innerhalb des Aneurysmas 4, dazu, dass das Embolisationsmittel 40 nach dem Platzieren nicht aus dem Aneurysma 4 entweichen kann, bis das Blut innerhalb des Aneurysmas 4 durch das Embolisationsmittel 40 geronnen und somit das Aneurysma 4 zuverlässig verschlossen ist.

Im Ausführungsbespiel gemäß Fig. 1 und in allen nachfolgenden Ausführungsformen ist die Abdeckung 20 als eine elektrogesponnene Abdeckung 20 ausgebildet.

Die Abdeckung 20 kann weiterhin auf einer Außenseite 30 und/oder auf einer Innenseite 32 der Gitterstruktur 14 angeordnet sein. Die Gitterstruktur 14 weist weiterhin Stege 34 auf oder ist aus ihnen gebildet, wobei die Stege 34 einstückig, also monolithisch, miteinander verbunden sind und geschlossene, insbesondere rautenförmige, Zellen 36 begrenzen. Das Blut fließt bei innerhalb des Gefäßes 6 platziertem medizinischen Set 2 somit durch die Zellen 36 hindurch und wird nicht gestaut. Die Stege 34 können auch, wie bereits beschrieben jeweils Stegpaare mit unterschiedlichen Stegbreiten aufweisen, um die Flexibilität der Gitterstruktur 14 zu erhöhen. Diese Ausbildung der Stegpaare mit unterschiedlichen Stegbreiten gilt nicht allein für dieses Ausführungsbeispiel gemäß Fig. 1 und ist somit nicht auf dieses Beschränkt. Vielmehr können alle im Rahmen dieser Anmeldung aufgeführten Ausführungsbeispiele die wie vorstehend beschrieben ausgebildeten Stegpaare aufweisen.

In Fig. 2 ist eine schematisierte Darstellung des erfindungsgemäßen medizinischen Sets 2 gemäß einer zweiten Ausführungsform gezeigt.

Im Wesentlichen und in Bezug auf den Hauptkatheter 8, die Gitterstruktur 14 sowie den Transportdraht 26 entspricht die zweite Ausführungsform der ersten Ausführungsform, welche in Fig. 1 beschrieben ist. Wesentlich für die zweite Ausführungsform ist, dass die Abdeckung 20 sich hierbei lediglich über einen Teil, insbesondere über höchstens 80%, insbesondere über höchstens 60% und speziell insbesondere über höchstens 40% der Länge L des zylinderförmigen Abschnitts 16 erstreckt.

Hierdurch ist erreicht, dass sich die Abdeckung 20 bei angeordneter Abdeckvorrichtung 12 lediglich innerhalb des Bereichs der Öffnung 28 des Aneurysmas 4 befindet, sodass an das Aneurysma 4 angrenzende Zellen und/oder Seitengefäße nicht von der Abdeckung 20 abgedeckt werden. Somit können die Zellen und/oder die Seitengefäße weiterhin aufgrund der mit Zellen 36 ausgebildeten Gitterstruktur 14 mit Blut und somit mit Nährstoffen versorgt werden.

In Fig. 3 ist eine dritte Ausführungsform des erfindungsgemäßen medizinischen Sets 2 schematisiert dargestellt. Analog zu den Ausführungen zum zweiten Ausführungsbeispiel wird hierbei ebenfalls lediglich auf die Unterschiede bezüglich der Abdeckung 20 eingegangen, da alle anderen Ausgestaltungen, vorzugsweise komplett, insbesondere im Wesentlichen, der Ausführungsform gemäß Fig. 2 oder der Ausführungsform gemäß Fig. 1 entsprechen. Dies betrifft insbesondere die Ausgestaltung des Hauptkatheters 8, des Transportdrahts 26 sowie die Ausgestaltung der Gitterstruktur 14.

Gemäß der dritten Ausführungsform erstreckt sich die Abdeckung 20 lediglich über einen Teil des Umfangs der Gitterstruktur 14 und über lediglich einen Teil der Länge L der Gitterstruktur 14. Speziell erstreckt sich hierbei die Abdeckung 20 insbesondere über höchstens 50%, insbesondere über höchstens 40% oder insbesondere über höchstens 30%, des Umfangs des zylinderförmigen Abschnitts 16 der Gitterstruktur 14.

Durch die dritte Ausführungsform ist eine Versorgung von dem Aneurysma 4 benachbarten Zellen und/oder Seitengefäßen weiterhin optimiert, da hierdurch bevorzugt lediglich die Öffnung 28 des Aneurysmas 4 abgedeckt ist und auch sich auf einer gleichen Höhe wie das Aneurysma 4 befindliche Zellen und/oder Seitengefäße weiterhin mit Blut und Nährstoffen versorgbar sind. Somit kann beispielsweise ein Seitengefäß, welches gegenüber des Aneurysmas 4 angeordnet ist, mit Blut versorgt werden.

Wie in Fig. 1 bis Fig. 3 gut zu sehen ist, sind bei dem medizinischen Set 2 und speziell bei der Gitterstruktur 14 Röntgenmarker 56 vorgesehen. Die Röntgenmarker 56 sind an Zellenspitzen der randseitigen Zellen 36 der Gitterstruktur 14 angeordnet. Konkret können die Röntgenmarker 56 als röntgensichtbare Hülsen, beispielsweise aus Platin oder Gold, gebildet sein, die auf die Zellenspitzen der randseitigen Zellen 36 aufgecrimpt sind.

Weiterhin ist durch die Expandierbarkeit der Gitterstruktur 14 und auch durch die poröse Ausgestaltung der Abdeckung 20 die Abdeckvorrichtung 12 für einen Einsatz mittels eines als Mikrokatheter ausgebildeten Hauptkatheters 8 ausgebildet.

Ergänzend sei angemerkt, dass die vorstehend genannten Ausführungsformen, insbesondere hinsichtlich eines sich Erstreckens der Abdeckung 20 entlang einer Länge L der Gitterstruktur 14 und hinsichtlich eines sich Erstreckens der Abdeckung 20 entlang eines Umfangs der Gitterstruktur 14 beliebig kombiniert ausgestaltet und kombiniert werden können. So ist beispielsweise auch eine Ausgestaltung möglich, bei der sich die Abdeckung 20 über die gesamte Länge L der Gitterstruktur erstreckt, sich jedoch zudem lediglich über einen Teil des Umfangs der Gitterstruktur 14 erstreckt.

Fig. 4 zeigt eine schematisierte Darstellung eines erfindungsgemäßen medizinischen Systems gemäß einer ersten Ausführungsform.

Das medizinische System weist hierbei das bereits vorstehend erwähnte medizinische Set 2 mit dem Hauptkatheter 8 sowie der Abdeckvorrichtung 12, die die Gitterstruktur 14 und die Abdeckung 20 aufweist, auf. Die Ausgestaltung der Abdeckung 20 entspricht hierbei der bereits vorstehend erwähnten zweiten Ausführungsform.

Weiterhin weist das medizinische System ein Embolisationsmittel 40 auf welches beispielsweise durch einen plastisch verformbaren Draht 42 oder durch eine Flüssigkeit gebildet ist. Das Embolisationsmittel 40 wird mittels eines Zusatzkatheters 44, welcher ebenfalls Teil des medizinischen Systems ist, innerhalb des Aneurysmas 4 platziert.

Der Zusatzkatheters 44 weist einen proximalen Bereich 46, einen Mittenbereich 48 sowie einen distalen Bereich 50 auf.

Zum Platzieren des Embolisationsmittels 40 ist der Zusatzkatheter 44 gemäß der ersten Ausführungsform des medizinischen Systems im Wesentlichen parallel, also neben dem medizinischen Set 2 innerhalb des Gefäßes 6 angeordnet. Hierbei wird dann der Zusatzkatheter 44 und speziell der distale Bereich 50 zwischen einer Gefäßwand und der Abdeckung 20 in das Aneurysma 4 "geschoben", um dort das Embolisationsmittel 40 in Form des plastisch verformbaren Drahtes 42 (auch als "Coil" bezeichnet) zu platzieren. Die Abdeckung 20 verhindert währenddessen und auch anschließend, dass das Embolisationsmittel 40 beispielsweise aufgrund der Blutströmung aus dem Aneurysma 4 heraustritt.

In Fig. 5 ist eine zweite Ausführungsform des medizinischen Systems schematisch dargestellt. Hierbei unterscheidet sich lediglich eine Anordnung des Zusatzkatheters 44 beim Platzieren des Embolisationsmittels 40 innerhalb des Aneurysmas 4 bzw. in das Aneurysma 4.

Gemäß der zweiten Ausführungsform ist der proximale Bereich 46 des Zusatzkatheters 44 im Wesentlichen parallel, also neben dem Hauptkatheter 8 geführt, während der Mittenbereich 48 durch die Abdeckvorrichtung 12 und speziell durch die Gitterstruktur 14 in das Innere der Gitterstruktur 14 geführt ist. Dies erfolgt insbesondere dadurch, dass der Inkreisdurchmesser D der Zellen 36 der Gitterstruktur 14 im expandierten Zustand wenigstens dem Außendurchmesser A des Zusatzkatheters 44 entspricht (hier nicht maßstabsgetreu dargestellt).

Somit ist ein einfaches Einführen des Zusatzkatheters 44 und speziell des Mittelbereiches 48 in die Gitterstruktur 14 erreicht. Weiterhin ist der distale Bereich 50 durch die Gitterstruktur 14 heraus und innerhalb des Aneurysmas 4 platziert. Zusätzlich ist der distale Bereich 50 des Zusatzkatheters 44 auch durch die Abdeckung 20 hindurchgeführt. Zur und nach der Platzierung des Embolisationsmittels 40 im Aneurysma 4 verhindert die Abdeckung 20 ebenfalls auch bei dem medizinischen System gemäß der zweiten Ausführungsform ein Heraustreten des Embolisationsmittels 40 aus dem Aneurysma 4.

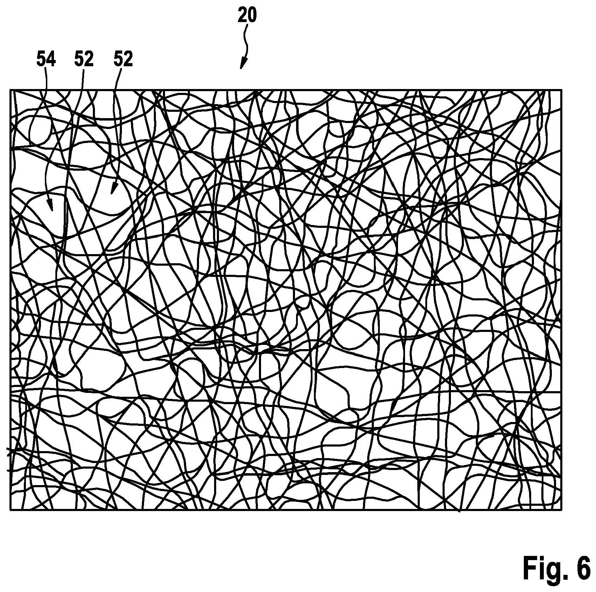

Die Gestaltung der Abdeckung 20 ist in der Rasterelektronenmikroskopaufnahme gemäß Fig. 6 gut erkennbar. Darin ist zu sehen, dass die Abdeckung 20 mehrere unregelmäßig große Poren 52 aufweist, die jeweils durch Fäden 54 begrenzt sind. Durch den Elektrospinnprozess werden mehrere Fäden 54 gebildet, die unregelmäßig zueinander ausgerichtet sind. Dabei bilden sich die Poren 52. Erkennbar ist in Fig. 6 auch, dass die Poren 52 eine vergleichsweise kleine Porengröße aufweisen, wobei einige Poren 52 jedoch ausreichend groß sind, um beispielsweise eine Blutdurchlässigkeit zu gewährleisten. Konkret sind in Fig. 6 vier Poren 52 grafisch hervorgehoben, die eine Größe von mehr als 30 µm² aufweisen. Die Dichte der Poren 52 mit einer Größe von mehr als 30 µm² lässt erkennen, dass die Abdeckung auf einer Fläche von 100.000 µm² wenigstens 10 derartiger Poren 52 aufweist.

In Fig. 6 ist jeweils auch erkennbar, dass sich die Fäden 54 der Abdeckung 20 mehrfach kreuzen. Eine Besonderheit des Elektrospinnverfahrens ist es jedoch, dass bei der Abdeckung 20 Stellen vorliegen, an welchen sich ausschließlich, d.h. nicht mehr als, zwei Fäden 54 überkreuzen. Daraus ist ersichtlich, dass die Abdeckung 20 insgesamt eine sehr dünne Wandstärke aufweist und daher hochflexibel ist.

Die hohe Flexibilität der Abdeckung 20 in Kombination mit der hohen Flexibilität der Gitterstruktur 14 führt dazu, dass eine Abdeckvorrichtung 12 bereitgestellt werden kann, die durch sehr kleine Zuführkatheter in ein (Blut-)Gefäß 6 eingeführt werden kann. Insbesondere können Zuführkatheter eingesetzt werden, die eine Größe von 6 French, insbesondere höchstens 5 French, insbesondere höchstens 4 French, insbesondere höchstens 3 French, insbesondere höchstens 2 French, aufweisen. Konkret kann die Abdeckvorrichtung nach den hier beschriebenen Ausführungsbeispielen bei Kathetern eingesetzt werden, die einen Innendurchmesser von höchstens 1,6 mm, insbesondere höchstens 1,0 mm, insbesondere höchstens 0,7 mm, insbesondere höchstens 0,4 mm aufweisen.

Die Schichtdicke der Abdeckung 20 beträgt in besonders bevorzugten Varianten höchstens 10 µm, insbesondere höchstens 8 µm, insbesondere höchstens 6 µm, insbesondere höchstens 4 µm. Dabei überkreuzen sich höchstens 4, insbesondere höchstens 3, insbesondere höchstens 2, Fäden 54. Generell sind innerhalb der elektrogesponnenen Struktur der Abdeckung 20 Kreuzungspunkte vorgesehen, in welchen sich nur 2 Fäden 54 überkreuzen. Die Gitterstruktur 10 weist vorzugsweise einen Querschnittsdurchmesser zwischen 2,5 mm und 8 mm, insbesondere zwischen 4,5 mm und 6 mm, auf.

### Bezugszeichenliste

- 2: medizinisches Set
- 4: Aneurysma
- 6: Gefäß
- 8: Hauptkatheter
- 10: Behandlungsort
- 12: Abdeckvorrichtung
- 14: Gitterstruktur
- 16: zylinderförmiger Abschnitt
- 18: distales Längsende
- 20: Abdeckung
- 22: trichterförmiger Abschnitt
- 24: proximales Längsende
- 26: Transportdraht
- 27: Umfangsfläche
- 28: Öffnung des Aneurysmas
- 30: Außenseite
- 32: Innenseite
- 34: Stege
- 36: Zelle
- 40: Embolisationsmittel
- 42: verformbarer Draht
- 44: Zusatzkatheter
- 46: proximaler Bereich
- 48: Mittenbereich
- 50: distaler Bereich
- 52: Pore
- 54: Faden
- 56: Röntgenmarker

- A: Außendurchmesser
- D: Inkreisdurchmesser
- F: Fließrichtung
- L: Länge

## Patentansprüche

1. Medizinisches Set (2) zur Behandlung von Aneurysmen (4) mit einem Hauptkatheter (8), einer durch den Hauptkatheter (8) hindurch an einen Behandlungsort (10) bewegbaren Abdeckvorrichtung (12) zum temporären Abdecken eines Aneurysmas (4), wobei die Abdeckvorrichtung (12) eine selbstexpandierbare Gitterstruktur (14) umfasst, die einen zylinderförmigen Abschnitt (16), der an einem distalen Längsende (18) offen und wenigstens teilweise mit einer Abdeckung (20) versehen ist, und einen trichterförmigen Abschnitt (22) aufweist, der dauerhaft mit einem innerhalb des Hauptkatheters (8) verschiebbaren Transportdraht (26) verbunden und über eine gesamte Umfangsfläche (27) abdeckungsfrei ist derart, dass die Gitterstruktur (14) in einem expandierten Zustand längsaxial blutdurchströmbar ist,
**dadurch gekennzeichnet, dass**
die Abdeckung (20) als eine elektrogesponnene Abdeckung (20) ausgebildet ist und auf einer Fläche von 100.000 µm² mindestens 10 Poren (52) umfasst, die eine Größe von mindestens 15 µm² aufweisen.

2. Medizinisches Set (2) nach Anspruch 1,
**dadurch gekennzeichnet, dass**
die Abdeckung (20) porös, insbesondere blutdurchlässig, ist oder dass die Abdeckung (20) derart geringfügig porös ist, sodass sie blutundurchlässig ist.

3. Medizinisches Set (2) nach Anspruch 1 oder 2,
**dadurch gekennzeichnet, dass**
die Abdeckung (20) eine Porosität von höchstens 70%, insbesondere höchstens 50%, aufweist und/oder dass
die Abdeckung (20) eine Porosität von wenigstens 5%, insbesondere wenigstens 10%, insbesondere wenigstens 20%, insbesondere wenigstens 30%, insbesondere wenigstens 40%, insbesondere wenigstens 45%, aufweist.

4. Medizinisches Set (2) nach einem der vorhergehenden Ansprüche,
**dadurch gekennzeichnet, dass**
sich die Abdeckung (20) über den gesamten Umfang des zylinderförmigen Abschnitts (16) erstreckt oder dass sich die Abdeckung (20) über einen Teil, insbesondere höchstens 50%, insbesondere höchstens 40%, insbesondere höchstens 30%, eines Umfangs der zylinderförmigen Abschnitts (16) erstreckt.

5. Medizinisches Set (2) nach einem der vorhergehenden Ansprüche,
**dadurch gekennzeichnet, dass**
sich die Abdeckung (20) über mindestens 80%, insbesondere über mindestens 90%, insbesondere über 100%, einer Länge (L) des zylinderförmigen Abschnitts (16) erstreckt und/oder
dass sich die Abdeckung (20) über höchstens 80%, insbesondere über höchstens 60%, insbesondere über höchstens 40%, der Länge (L) des zylinderförmigen Abschnitts (16) erstreckt, wobei die Abdeckung (20) vom distalen Ende (18) des zylinderförmigen Abschnitts (16) beabstandet ist.

6. Medizinisches Set (2) nach einem der vorhergehenden Ansprüche,
**dadurch gekennzeichnet, dass**
die Abdeckung (20) durch ein Kunststoffmaterial, insbesondere ein Polymer, vorzugsweise Polyurethan, gebildet ist und/oder
dass die Abdeckung (20) auf einer Außenseite (30) und/oder einer Innenseite (32) der Gitterstruktur (14) angeordnet ist.

7. Medizinisches Set (2) nach einem der vorhergehenden Ansprüche,
**dadurch gekennzeichnet, dass**
die Gitterstruktur (14) aus Stegen (34) gebildet ist, die einstückig miteinander verbunden sind und geschlossene, insbesondere rautenförmige, Zellen (36) begrenzen.

8. Medizinisches System zur Behandlung von Aneurysmen (4) mit einem medizinischen Set (2) nach einem der vorhergehenden Ansprüche, wobei das Set (2) aufweist:
- den Hauptkatheter (8),
- die durch den Hauptkatheter (8) hindurch an einen Behandlungsort (10) bewegbare Abdeckvorrichtung (12) zum temporären Abdecken des Aneurysmas (4) und
- wenigstens ein Embolisationsmittel (40) zur Platzierung im Aneurysma (4).

9. Medizinisches System nach Anspruch 8,
**dadurch gekennzeichnet, dass**
ein Zusatzkatheter (44) mit einem proximalen Bereich (46), einem Mittenbereich (48) sowie mit einem distalen Bereich (50), zur Zuführung des Embolisationsmittels (40) in das Aneurysma (4) vorgesehen ist, wobei der Zusatzkatheter (44) vom Hauptkatheter (8) unabhängig und/oder gegenüber dem Hauptkatheter (8) relativbeweglich ist.

10. Medizinisches System nach dem vorhergehenden Anspruch,
**dadurch gekennzeichnet, dass**
der Mittenbereich (48) des Zusatzkatheters (44) innerhalb der Abdeckvorrichtung (12) und der distale Bereich (50) außerhalb der Abdeckvorrichtung (12), insbesondere im Aneurysma (4), sowie der proximale Bereich (46) parallel zum Hauptkatheter (8) angeordnet ist.

11. Medizinisches System nach Anspruch 9,
**dadurch gekennzeichnet, dass**
der proximale Bereich (46) parallel zum Hauptkatheter (8) angeordnet ist und der Mittenbereich (48) und der distale Bereich (50) außerhalb der Abdeckvorrichtung (12) angeordnet sind, wobei das distale Ende (50) insbesondere im Aneurysma (4) angeordnet ist.

12. Medizinisches System (2) nach einem der Ansprüche 9 bis 11,
**dadurch gekennzeichnet, dass**
die Zellen (36) der Gitterstruktur (14) im expandierten Zustand einen Inkreisdurchmesser (D) aufweisen oder auf einen Inkreisdurchmesser (D) aufweitbar sind, der wenigstens dem Außendurchmesser (A) des Zusatzkatheters (44) entspricht.

13. Medizinisches System (2) nach einem der Ansprüche 8 bis 12,
**dadurch gekennzeichnet, dass**
das Embolisationsmittel (40) durch einen plastisch verformbaren Draht (42), insbesondere einen Coil, oder durch eine Flüssigkeit gebildet ist.

## Claims

1. Medical kit (2) for the treatment of aneurysms (4) with a main catheter (8), a covering device (12) that is movable through the main catheter (8) to a treatment location (10) for the temporary covering of an aneurysm (4), wherein the covering device (12) comprises a self-expanding lattice structure (14) which has cylindrical section (16) which is open at a distal longitudinal end (18) and is at least partially provided with a cover (20), and a funnel-shaped section (22) which is permanently connected to a displaceable transport wire (26) inside the main catheter (8) and over an entire circumferential area (27) is cover-free, so that in the expanded state the lattice structure (14) is blood-permeable in a longitudinal direction,
**characterised in that**
the cover (20) is designed as an electro-spun cover (20) and comprises at least 10 pores (52) with a size of at least 15 µm² over an area of 100,000 µm².

2. Medical kit (2) according to claim 1,
**characterised in that**
the cover (20) is porous, more particularly blood-permeable, or **in that** the cover (20) is porous to such a small degree that is it impermeable to blood.

3. Medical kit (2) according to claim 1 or 2,
**characterised in that**
the cover (20) has a porosity of at most 70 %, more particularly at most 50 %, and/or **in that** the cover (20) has a porosity of at least 5 %, more particularly at least 10 %, more particularly at least 20 %, more particularly at least 30 %, more particularly at least 40 %, more particularly at least 45 %.

4. Medical kit (2) according to any one of the preceding claims,
**characterised in that**
the cover (20) extends over the entire circumference of the cylindrical section (16) or **in that** the cover (20) extends over a part, more particularly at most 50 %, more particularly at most 40 %, more particularly at most 30 %, of a circumference of the cylindrical section (16).

5. Medical kit (2) according to any one of the preceding claims,
**characterised in that**
the cover (20) extends over at least 80 %, more particularly over at least 90 %, more particularly over 100 %, of a length (L) of the cylindrical section (16) and/or **in that** the cover (20) extends over at most 80 %, more particularly over at most 60 %, more particularly over at most 40 %, of the length (L) of the cylindrical section (16), wherein the cover (20) is spaced apart from the distal end (18) of the cylindrical section (16).

6. Medical kit (2) according to any one of the preceding claims,
**characterised in that**
the cover (20) is made of a plastic material, more particularly a polymer, preferably polyurethane, and/or in that the cover (20) is arranged on an outer side (30) and/or an inner side (32) of the lattice structure (14).

7. Medical kit (2) according to any one of the preceding claims,
**characterised in that**
the lattice structure (14) is formed of struts (34) which are connected in one piece with each other and define closed, more particularly diamond-shaped, cells (36).

8. Medical system for the treatment of aneurysms (4) with a medical kit (2) according to any one of the preceding claims, wherein the kit (2) comprises:
- the main catheter (8),
- the covering device (12) which can be moved through the main catheter (8) to a treatment location (10) for the temporary covering of the aneurysm (4) and
- at least one embolisation agent (40) for placement in the aneurysm (4).

9. Medical system according to claim 8,
**characterised in that**
an additional catheter (44) is provided with a proximal area (46), a middle area (48) as well as with a distal area (50) for supplying the embolisation agent (40) into the aneurysm (4), wherein the additional catheter (44) is relatively displaceable independently of the main catheter (8) and/or with regard to the main catheter (8).

10. Medical system according to the preceding claim,
**characterised in that**
the middle area (48) of the additional catheter (44) is arranged inside the covering device (12) and the distal area (50) outside the covering device (12), more particularly in the aneurysm (4), and the proximal area (46) in parallel to the main catheter (8).

11. Medical system according to claim 9,
**characterised in that**
the proximal area (46) is arranged in parallel to the main catheter (8) and the middle area (48) and the distal area (50) are arranged outside the covering device (12), in particular wherein the distal end (50) isarranged in the aneurysm (4).

12. Medical system (2) according to any one of claims 9 to 11,
**characterised in that**
in the expanded state, the cells (36) of the lattice structure (14) have an inner circumferential diameter (D) or can be expanded to an inner circumferential diameter (D) which at least corresponds to the outer diameter (A) of the additional catheter (44).

13. Medical system (2) according to any one of claims 8 to 12,
**characterised in that**
the embolisation agent (40) is formed by a plastically deformable wire (42), more particularly a coil, or by a fluid.

## Revendications

1. Kit médical (2) pour le traitement des anévrismes (4) avec un cathéter principal (8), un dispositif de couverture mobile à travers le cathéter principal (8) vers un dispositif de couverture (12) déplaçable vers un lieu de traitement (10) pour couvrir temporairement un anévrisme (4), le dispositif de couverture (12) comprenant une structure en treillis auto-extensible (14) comprenant une section cylindrique (16) ouverte à une extrémité distale (18) et dotée au moins partiellement d'un couvercle (20), et comportant une section en forme d'entonnoir (22), reliée en permanence à un fil de transport (26) déplaçable à l'intérieur du cathéter principal (8) et sans couverture sur toute une surface circonférentielle (27) de telle sorte que la structure de la grille (14), dans un état expansé, soit perméable à la circulation sanguine dans le sens axial de la longueur,
**caractérisé en ce que**
le couvercle (20) est conçu comme un couvercle électrofilé (20) et comprend sur une surface de 100 000 µm² au moins 10 pores (52) qui présentent une taille minimale de 15 µm².

2. Kit médical (2) selon la revendication 1,
**caractérisé en ce que**
la couverture (20) est poreuse, en particulier perméable à la circulation sanguine, ou que la couverture (20) est légèrement poreuse au point d'être imperméable à la circulation sanguine.

3. Ensemble médical (2) selon la revendication 1 ou 2,
**caractérisé en ce que**
la couverture (20) présente une porosité maximale de 70 %, en particulier de 50 %, et/ou que la couverture (20) présente une porosité minimale de 5 %, en particulier 10 %, en particulier 20 %, en particulier 30 %, en particulier 40 %, en particulier 45 %.

4. Ensemble médical (2) selon l'une des revendications précédentes,
**caractérisé en ce que**
la couverture (20) s'étend sur l'ensemble du périmètre de la section cylindrique (16) ou **en ce que** la couverture (20) s'étend sur une partie, en particulier au maximum 50 %, en particulier au maximum 40 %, en particulier au maximum 30 %, d'un périmètre de la section cylindrique (16).

5. Ensemble médical (2) selon l'une des revendications précédentes,
**caractérisé en ce que**
la couverture (20) s'étend sur au moins 80 %, en particulier sur au moins 90 %, en particulier sur 100 %, d'une longueur (L) de la section cylindrique (16) et/ou que la couverture (20) s'étend au maximum sur 80 %, en particulier sur au maximum 60 %, en particulier sur au maximum 40 %, de la longueur (L) de la section cylindrique (16), le couvercle (20) étant espacé de l'extrémité distale (18) de la section cylindrique (16).

6. Ensemble médical (2) selon l'une des revendications précédentes,
**caractérisé en ce que**
le couvercle (20) est formé par une matière plastique, en particulier un polymère, de préférence un polyuréthane, et/ou que le couvercle (20) est placé sur une face extérieure (30) et/ou une face intérieure (32) de la structure en treillis (14).

7. Ensemble médical (2) selon l'une des revendications précédentes,
**caractérisé en ce que**
la structure en treillis (14) est formée de traverses (34) qui sont connectées en un seul bloc et délimitent des cellules fermées, en particulier en forme de losanges (36).

8. Système médical pour le traitement des anévrismes (4) avec un kit médical (2) selon l'une des revendications précédentes, l'ensemble (2) comportant :
- le cathéter principal (8),
- le dispositif de couverture mobile (12) traversant le cathéter principal (8) jusqu'à un lieu de traitement (10) pour couvrir temporairement l'anévrisme (4) ; et
- au moins un agent embolisant (40) destiné à être placé dans l'anévrisme (4).

9. Système médical selon la revendication 8,
**caractérisé en ce que** :
un cathéter additionnel (44) avec une zone proximale (16), une zone centrale (48) et une zone distale (50) est prévu pour l'introduction de l'agent embolisant (40) dans l'anévrisme (4), le cathéter additionnel (44) étant indépendant du cathéter principal (8) et/ou relativement mobile par rapport au cathéter principal (8).

10. Système médical selon la revendication précédente,
**caractérisé en ce que**
la zone centrale (48) du cathéter additionnel (44) est située à l'intérieur du dispositif de couverture (12) et la zone distale (50) à l'extérieur du dispositif de couverture (12), en particulier dans l'anévrisme (4), et que la zone proximale (46) est disposée de manière à être parallèle au cathéter principal (8).

11. Système médical selon la revendication 9,
**caractérisé en ce que**
la zone proximale (46) est disposée parallèlement au cathéter principal (8) et que la zone centrale (48) et la zone distale (50) sont disposées en dehors du dispositif de couverture (12), l'extrémité distale (50) étant située en particulier dans l'anévrisme (4).

12. Système médical (2) selon l'une des revendications 9 à 11,
**caractérisé en ce que**
les cellules (36) de la structure en treillis (14), à l'état expansé, ont un diamètre de cercle intérieur (D) ou sont extensibles jusqu'à un diamètre de cercle intérieur (D) au moins égal au diamètre extérieur (A) du cathéter additionnel (44).

13. Système médical (2) selon l'une des revendications 8 à 12,
**caractérisé en ce que**
l'agent embolisant (40) est formé par un fil plastique déformable (42), en particulier une bobine, ou par un liquide.
